# EUROPEAN PATENT SPECIFICATION

(11) **EP 2 018 419 B1**
(45) Date of publication and mention of the grant of the patent: **04.05.2016**
(21) Application number: 06769014.9
(22) Date of filing: 23.06.2006
(51) Int. Cl.: C12N 5/00

(54) **SEPARATION METHOD OF NUCLEATED CELLS DERIVED FROM BONE MARROW FOR BONE FORMATION**
TRENNVERFAHREN FÜR AUS KNOCHENMARK STAMMENDE KERNHALTIGE ZELLEN ZUR KNOCHENBILDUNG
PROCÉDÉ DE SÉPARATION DE CELLULES NUCLÉÉES DÉRIVÉES DE MOELLE OSSEUSE UTILISÉES POUR LA FORMATION OSSEUSE

(30) Priority: 16.05.2006 KR 20060043936
(43) Date of publication of application: 28.01.2009
(73) Proprietor: Sewon Cellontech Co., Ltd., Youngdeungpo-Gu, Seoul 150-712 (KR)
(72) Inventor: PARK, Hyun-Shin, Seoul 158-076 (KR); JANG, Jae-Deog, Seoul 139-784 (KR); LEE, Sae-Bom, Seoul 143-210 (KR); CHANG, Cheong-Ho, Seoul 137-040 (KR)
(74) Representative: von Kreisler Selting Werner - Partnerschaft von Patentanwälten und Rechtsanwälten mbB
(86) International application number: PCT/KR2006/002434
(87) International publication number: WO 2007/132962

(56) References cited:
- WO-A1-97/26326
- WO-A2-03/040064
- KR-A- 20030 085 257
- US-A- 5 824 084
- US-A1- 2002 086 344
- US-A1- 2005 026 297
- US-B1- 6 673 618
- IM ET AL: "Do adipose tissue-derived mesenchymal stem cells have the same osteogenic and chondrogenic potential as bone marrow-derived cells?" OSTEOARTHRITIS AND CARTILAGE, BAILLIERE TINDALL, LONDON, GB LNKD- DOI:10.1016/J.JOCA.2005.05.005, vol. 13, no. 10, 1 October 2005 (2005-10-01), pages 845-853, XP005094309 ISSN: 1063-4584
- GANGJI V ET AL: "STEM CELL THERAPY FOR OSTEONECROSIS OF THE FEMORAL HEAD" EXPERT OPINION ON BIOLOGICAL THERAPY, ASHLEY, LONDON, GB LNKD- DOI:10.1517/14712598.5.4.437, vol. 5, no. 4, 1 April 2005 (2005-04-01), pages 437-442, XP009066384 ISSN: 1471-2598
- NOEL D ET AL: "REGENERATIVE MEDICINE THROUGH MESENCHYMAL STEM CELLS FOR BONE AND CARTILAGE REPAIR" CURRENT OPINION IN INVESTIGATIONAL DRUGS, PHARMAPRESS, US, vol. 3, no. 7, 1 July 2002 (2002-07-01), pages 1000-1004, XP008019406 ISSN: 1472-4472

## Description

### Technical Field

The present invention relates to a method for separating bone marrow-derived nucleated cells for bone formation, and more particularly to a method allowing the rapid and convenient separation of bone marrow-derived nucleated cells, which can be grafted into a site in need of bone formation or bone defect treatment. According to the inventive method, bone formation in emergency patients or patients undergoing repeated surgical operations can be effectively achieved by separating the nucleated cells for bone formation in surgical locations in a convenient and rapid manner and injecting the separated cells into the patients.

### Background Art

According to a report by WHO, more than 50% of the old-age population aged 65 years or older suffer from chronic bone diseases, and the rate of bone fractures caused by osteoporosis increased by more than two times over the past ten years. It corresponds to more than about 40% of the female population aged 50 years or older. In USA, about 5,600,000 persons per year suffer a bone fracture, 3,100,000 persons of which undergo a surgical operation. According to statistical data of medical data international (MDI), about 426,000 cases of bone grafting were performed. The cost used for bone grafting is about eight hundred million dollars per year worldwide, and in the year 1995, autografting, allografting and synthetic material grafting were performed at a ratio of about 58%: 34%: 8%.

Usually, a simple fracture is sufficiently healed only with casting for several weeks, but in the case of a severe fracture or bone defect, bone grafting operations have been performed. However, autografting causes a severe pain at a harvest site, requires a long recovery period and has a difficulty in obtaining a bone graft donor site. Allografting is very limited in its use due to the weakening of bone strength caused by sterilization, an immune rejection response, resorption and a refracture and has a fatal shortcoming in that there is a possibility of infection such as hepatitis or AIDS. For this reason, the use of grafting has been reduced since the latter part of 1993 after Food & Drug Administration tightened regulations for a bone tissue bank on infection.

In another bone grafting technique, for example, a metal coated with a biologically active or inactive ceramic is used as a support in orthopedic surgery. However, the use of metal as a bone graft material has many difficulties due to problems such as the corrosion of metal, the surface abrasion of ceramic-metal and the formation of severe fibrous tissue on bone and graft surfaces.

Regarding bone morphogenic factors, various factors have been studied since Urist and Mclean presented bone morphogenesis of bone morphogenic proteins in 1952. However, these factors have a limitation on their general clinical application, because these are inefficient in that these are produced using a complex and expensive process in small amounts.

Bone marrow injection is based on the reports of Huggins (1931), Friedenstein (1973), Ashton (1980) and the like, which indicate that osteoprogenitor cells in bone marrow induce and promote bone formation. This bone marrow injection is performed mainly for bone fracture healing alone or in combination with bone grafting. Unlike other bone grafting techniques, it does not perform the skin incision of a donor site, and thus has no problem associated with the donor site. In addition, it has no complications or side effects.

Since then, although good results through the clinical application of the bone marrow injection have also been reported, a significant portion of the results are not constant. Also, the theoretical basis of the results is unclear in that the amount of bone marrow, which can be extracted from one site, is limited, and the number of osteoprogenitor cells contained in bone marrow is very limited.

Thus, it is very effective to use a novel bone grafting method for bone formation, which comprises amplifying and culturing osteoprogenitor cells into a sufficient number of osteoblasts and injecting the cultured osteoblasts into a bone formation site. Although this method has many advantages and effects compared to the existing au- tografting, allografting and bone marrow injection techniques, it has a problem in that, because it requires a culturing period of 3-4 weeks for the proliferation of osteoblasts, it is difficult to apply for emergency patients or patients who require repeated surgical operations.

### Disclosure of Invention

### Technical Problem

The present invention has been made to solve the above-described problems occurring in the prior art, and a first object of the present invention is to provide a method for separating bone marrow-derived nucleated cells for bone formation, which improves the problems of implantation, bone grafting, grafting of cultured osteoblasts, and the like. A second object of the present invention is to provide a method for separating bone marrow-derived nucleated cells for bone formation, which eliminates a clinical rejection response by extracting a small amount of bone marrow for bone formation, separating only nucleated cells for bone formation from the bone marrow, and the injecting the separated cells. A third object of the present invention is to provide a method for separating bone marrow-derived nucleated cells for bone formation, which achieves effective bone formation in emergency patients or patients undergoing repeated surgical operations, by separating nucleated cells for bone formation in surgical locations in a rapid and convenient manner and injecting the separated cells into the patients. A fourth object of the present invention is to provide a method for separating bone marrow-derived nucleated cells for bone formation, which allows effective bone formation to be induced by separating nucleated cells from the bone marrow extracted according to the present invention and injecting the separated cells into a bone formation site. In addition, a fifth object of the present invention is to provide a method for separating bone marrow-derived nucleated cells for bone formation, which greatly increase the reliability of cell separation, such that consumers can have a good image for the cell separation.

### Technical Solution

To achieve the above objects, the present invention provides a method for separating bone marrow-derived nucleated cells for bone formation, which comprises the steps of: washing bone marrow; lysing red blood cells in the bone marrow; neutralizing the bone marrow lysate; purifying nucleated cells from the neutralized bone marrow; and mixing the nucleated cells with a maintenance buffer for bone formation, as defined in claim 1.

### Best Mode for Carrying Out the Invention

Hereinafter, a preferred embodiment of the present invention will be described in further detail.

The construction of the inventive method for separating bone marrow-derived nucleated cells for bone formation is as follows.

In the following description, a detailed description of known functions and configurations incorporated herein will be omitted when it may make the subject matter of the present invention unclear.

Furthermore, the terms described below are established taking into account the functions of the present invention. Since the terms may be changed in accordance with manufacturer's intention or practice, the meanings of the terms should be defined based on the whole contents of the specification.

The inventive method comprises the steps of: washing bone marrow; lysing red blood cells in the bone marrow; neutralizing the bone marrow lysate; purifying nucleated cells from the neutralized bone marrow; and mixing the nucleated cells with a maintenance buffer for bone formation, and the technical construction thereof is as follows.

The step of washing bone marrow comprises the sub-steps of: extracting 2-5 ml of bone marrow from a patient and storing the extracted bone marrow in a washing buffer; shaking the bone-marrow washing buffer so as to sufficiently remove impurities or fatty components from the bone marrow and centrifuging the shaken washing buffer at 1,600 rpm for 4-5 minutes; removing the supernatant, transferring the remaining bone marrow component into another washing container, shaking the bone marrow-containing container so as to achieve sufficient mixing, and then centrifuging the shaken solution at 1,600 rpm for 4-5 minutes; and removing the supernatant from the centrifuged solution.

Also, the step of lysing red blood cells comprises the sub-steps of: adding a 10-fold amount of a lysis buffer to the bone marrow; and mixing the lysis buffer with the bone marrow several times using a syringe and then standing the solution for 10-20 seconds.

Also, the step of neutralizing the bone marrow solution comprises the sub-steps of: mixing the bone marrow solution with the same amount of a neutralizing buffer; and centrifuging said solution at 1,600 rpm for 4-5 minutes and then removing the supernatant except for the precipitated nucleated cells for bone formation.

Furthermore, the step of purifying the nucleated cells comprises the sub-steps of: mixing 4-5 ml of a maintenance buffer with the nucleated cells in a container; and centrifuging said solution at 1,600 rpm for 4-5 minutes and then removing the supernatant.

In addition, the step of mixing the nucleated cells with the maintenance buffer comprises the sub-steps of: adding 2-3 ml of the maintenance buffer to the nucleated cells in view of a lesion and then floating the nucleated cells on the container bottom using a syringe; and placing the nucleated cells and the maintenance buffer into a syringe and then injecting the contents of the syringe into a bone defect site or a site in need of bone formation.

### Mode for the Invention

According to the method of the present invention, the bone marrow-derived nucleated cells for bone formation, which can be grafted into a site in need of bone defect treatment or bone formation, can be separated in a surgical location in a rapid and convenient manner. An example of the method according to the present invention is as follows.

### Example

First, a kit for separating bone marrow-derived nucleated cells for bone formation is prepared so as to have the following construction:
1) Two solutions for storing and washing extracted bone marrow;
2) Lysis buffer for red blood cells;
3) Neutralization buffer;
4) Maintenance buffer for bone formation; and
5) Bone grafting syringe

Using the above kit construction, the inventive method for separating bone marrow-derived nucleated cells for bone formation can be carried out in the following manner.
1) 2-5 ml of bone marrow is extracted from a patient and stored in the washing solution. In extracting the bone marrow from the iliac crest, the amount of pure bone marrow is about 2-5 ml, and an extract exceeding said amount corresponds to peripheral blood components. Thus, it is effective for the extraction of pure bone marrow to extract the bone marrow in an amount of 2-5 ml.
2) A container containing the bone marrow in the washing solution is shaken so as to sufficiently remove impurities or fatty components from the bone marrow, and then the solution is centrifuged at 1,600 rpm for 5 minutes. The centrifugation speed (rpm) and time are minimal conditions in which the cells are precipitated without being damaged.
3) After removing the supernatant, the bone marrow component remaining on the container bottom is transferred to a second washing solution, and the bone marrow-containing container is shaken so as to sufficiently mix the bone marrow with the second washing solution, and then the solution is centrifuged at 1,600 rpm for 5 minutes.
4) After removing the supernatant, a 10-fold amount of a lysis buffer for red blood cells is added to the remaining bone marrow solution. The 10-fold amount of the lysis buffer serves to lyse red blood cells in the bone marrow solution using osmotic pressure while minimizing damage to the nucleated cells.
5) The lysis buffer and the bone marrow solution are mixed several times with each other using a syringe and then left to stand for 10-20 seconds to achieve the lysis of red blood cells.
6) The same amount of a neutralization buffer is added to the bone marrow and then carefully shaken to mix the neutralization buffer with the bone marrow. The neutralization buffer used herein is a 10-fold concentrated buffer.
7) After said solution is centrifuged at 1,600 rpm for 5 minutes, the supernatant except for the precipitated nucleated cells for bone formation is removed.
8) A maintenance buffer for bone formation is added into the nucleated cell- containing container, and then the container is shaken several times.
9) The solution is centrifuged at 1,600 rpm for 5 minutes, and the supernatant is removed.
10) A maintenance buffer for bone formation is added to the cells in an amount selected in view of the size of a lesion, and then the cells on the container bottom are floated using a syringe.
11) The nucleated cells for bone formation and the maintenance buffer for bone formation are placed into the syringe and then injected into a bone defect site or a site in need of bone formation.

According to the above-described method, the nucleated cells for bone formation are separated from the bone marrow extracted from the patient and injected into the site in need of bone formation, and thus a clinical rejection response does not occur. Also, because the nucleated cells for bone formation are separated and grafted within a short time after the extraction of bone marrow, bone formation can be induced in an effective and rapid manner.

### Industrial Applicability

As described in detail above, the present invention aims to improve the problems occurring in implantation, bone grafting, grafting of cultured osteoblasts, and the like. Particularly, according to the present invention, a clinical rejection response is eliminated by extracting a small amount of bone marrow for bone formation, separating only nucleated cells for bone formation from the bone marrow, and the injecting the separated cells.

Also, according to the present invention, effective bone formation in emergency patients or patients undergoing repeated surgical operations is achieved by separating nucleated cells for bone formation in surgical locations in a rapid and convenient manner and injecting the separated cells into the patients.

In addition, according to the present invention, effective bone formation can be induced by separating nucleated cells from the bone marrow extracted according to the present invention and injecting the separated cells into a bone formation site.

As a result, according to the present invention, the reliability of cell separation can be greatly increased such that consumers can have a good image for the cell separation.

## Claims

1. A method for separating bone marrow-derived nucleated cells for bone formation, which comprises the steps of:
(a) washing bone marrow, comprising the sub-steps of:
(a1) storing 2-5 ml of bone marrow extracted from a patient in a washing buffer;
(a2) shaking the bone-marrow washing buffer 40-50 times so as to sufficiently remove impurities or fatty components from the bone marrow,
(a3) centrifuging the shaken washing buffer at 1,600 rpm for 4-5 minutes;
(a4) removing the supernatant,
(a5) transferring the remaining bone marrow component into another washing container,
(a6) shaking the bone marrow-containing container 40-50 times,
(a7) centrifuging the shaken solution at 1,600 rpm for 4-5 minutes, and
(a8) removing the supernatant from the centrifuged solution;
(b) lysing red blood cells in the bone marrow, comprising the sub-steps of:
(b1) adding a 10-fold amount of a lysis buffer to the bone marrow; and
(b2) mixing the lysis buffer with the bone marrow several times using a syringe, and
(b3) standing the solution for 10-20 seconds;
(c) neutralizing the bone marrow lysate, comprising the sub-steps of:
(c1) mixing the bone marrow solution with the same amount of a neutralizing buffer;
(c2) centrifuging said solution at 1,600 rpm for 4-5 minutes, and
(c3) removing the supernatant except for the precipitated nucleated cells for bone formation;
(d) purifying nucleated cells from the neutralized bone marrow, comprising the sub-steps of:
(d1) mixing 4-5 ml of a maintenance buffer with the nucleated cells in a container; and
(d2) centrifuging said solution at 1,600 rpm for 4-5 minutes, and
(d3) removing the supernatant; and
(e) mixing the nucleated cells with a maintenance buffer for bone formation.

2. The method of claim 1, wherein the step (e) of mixing the nucleated cells with the maintenance buffer comprises the sub-steps of:
(e1) adding 2-3 ml of the maintenance buffer to the nucleated cells in view of a lesion,
(e2) floating the nucleated cells on the container bottom using a syringe; and
(e3) placing the nucleated cells and the maintenance buffer into a syringe for injection into a bone defect site or a site in need of bone formation.

## Patentansprüche

1. Verfahren zum Abtrennen vom Knochenmark abgeleiteter kernhaltiger Zellen zur Knochenbildung, das die folgenden Schritte umfasst:
(a) Waschen von Knochenmark, umfassend die Teilschritte:
(a1) Aufbewahren von 2-5 ml Knochenmark, das einem Patienten entnommen wurde, in einem Waschpuffer;
(a2) 40-50-maliges Schütteln des Knochenmarks/Waschpuffers, um Verunreinigungen oder Fettkomponenten ausreichend aus dem Knochenmark zu entfernen;
(a3) Zentrifugieren des geschüttelten Waschpuffers mit 1600 U/min während 4-5 Minuten;
(a4) Entfernen des Überstands;
(a5) Überführen der restlichen Knochenmarkkomponente in einen anderen Waschbehälter;
(a6) 40-50-maliges Schütteln des knochenmarkhaltigen Behälters;
(a7) Zentrifugieren der geschüttelten Lösung mit 1600 U/min während 4-5 Minuten; und
(a8) Entfernen des Überstands aus der zentrifugierten Lösung;
(b) Lysieren der roten Blutkörperchen im Knochenmark, umfassend die Teilschritte:
(b1) Hinzufügen einer 10-fachen Menge eines Lysepuffers zu dem Knochenmark; und
(b2) mehrmaliges Mischen des Lysepuffers mit dem Knochenmark mit Hilfe einer Spritze; und
(b3) Stehenlassen der Lösung während 10-20 Sekunden;
(c) Neutralisieren des Knochenmarklysats, umfassend die Teilschritte:
(c1) Mischen der Knochenmarklösung mit derselben Menge eines neutralisierenden Puffers;
(c2) Zentrifugieren der Lösung mit 1600 U/min während 4-5 Minuten; und
(c3) Entfernen des Überstands außer den ausgefällten kernhaltigen Zellen für die Knochenbildung;
(d) Reinigen von kernhaltigen Zellen aus dem neutralisierten Knochenmark, umfassend die Teilschritte:
(d1) Mischen von 4-5 ml eines Erhaltungspuffers mit den kernhaltigen Zellen in einem Behälter; und
(d2) Zentrifugieren der Lösung mit 1600 U/min während 4-5 Minuten; und
(d3) Entfernen des Überstands; und
(e) Mischen der kernhaltigen Zellen mit einem Erhaltungspuffer zur Knochenbildung.

2. Verfahren gemäß Anspruch 1, wobei Schritt (e) des Mischens der kernhaltigen Zellen mit dem Erhaltungspuffer die folgenden Teilschritte umfasst:
(e1) Hinzufügen von 2-3 ml des Erhaltungspuffers zu den kernhaltigen Zellen in Anbetracht einer Läsion;
(e2) Aufwirbeln der kernhaltigen Zellen auf dem Behälterboden mit Hilfe einer Spritze; und
(e3) Einbringen der kernhaltigen Zellen und des Erhaltungspuffers in eine Spritze zur Injektion in eine Knochendefektstelle oder eine Stelle, die einer Knochenbildung bedarf.

## Revendications

1. Procédé pour séparer des cellules nucléées dérivées de la moelle osseuse pour la formation osseuse, comprenant les étapes consistant à :
(a) laver de la moelle osseuse, comprenant les sous-étapes consistant à :
(a1) conserver 2-5 ml de moelle osseuse extraite d'un patient dans un tampon de lavage,
(a2) secouer la moelle osseuse/le tampon de lavage 40-50 fois pour enlever suffisamment des impuretés ou des composants gras de la moelle osseuse,
(a3) centrifuger le tampon de lavage secoué à 1600 tpm pendant 4-5 minutes,
(a4) enlever le surnageant,
(a5) transférer le composant restant de la moelle osseuse dans une autre cuve de lavage,
(a6) secouer la cuve contenant la moelle osseuse 40-50 fois,
(a7) centrifuger la solution secouée à 1600 tpm pendant 4-5 minutes, et
(a8) enlever le surnageant de la solution centrifugée,
(b) lyser des globules rouges dans la moelle osseuse, comprenant les sous-étapes consistant à :
(b1) ajouter une quantité dix fois supérieure d'un tampon de lyse à la moelle osseuse, et
(b2) mélanger le tampon de lyse avec la moelle osseuse plusieurs fois à l'aide d'une seringue, et
(b3) laisser reposer la solution pendant 10-20 secondes,
(c) neutraliser le lysat de moelle osseuse, comprenant les sous-étapes consistant à :
(c1) mélanger la solution de moelle osseuse avec la même quantité d'un tampon neutralisant,
(c2) centrifuger ladite solution à 1600 tpm pendant 4-5 minutes, et
(c3) enlever le surnageant sauf pour les cellules nucléées précipitées pour la formation osseuse,
(d) purifier des cellules nucléées de la moelle osseuse neutralisée, comprenant les sous-étapes consistant à :
(d1) mélanger 4-5 ml d'un tampon d'entretien avec les cellules nucléées dans un récipient, et
(d2) centrifuger ladite solution à 1600 tpm pendant 4-5 minutes, et
(d3) enlever le surnageant, et
(e) mélanger les cellules nucléées avec un tampon d'entretien pour la formation osseuse.

2. Procédé selon la revendication 1, dans lequel l'étape (e) consistant à mélanger les cellules nucléées avec le tampon d'entretien comprend les sous-étapes consistant à :
(e1) ajouter 2-3 ml du tampon d'entretien aux cellules nucléées, compte tenu d'une lésion,
(e2) faire flotter les cellules nucléées sur le fond du récipient à l'aide d'une seringue, et
(e3) placer les cellules nucléées et le tampon d'entretien dans une seringue pour injection dans un site avec défaut osseux ou dans un site nécessitant de la formation osseuse.
